# EUROPEAN PATENT APPLICATION

(11) **EP 3 053 544 A1**
(43) Date of publication of application: **10.08.2016**
(21) Application number: 15154328.7
(22) Date of filing: 09.02.2015
(51) Int. Cl.: A61F 2/00, A61J 1/14, A61L 2/00, A61L 2/025, B65B 55/02, A61B 90/00, B08B 3/04, B08B 3/12, A61F 2/32, A61F 2/38, B65D 81/00

(54) **CONTAINER FOR AN IMPLANT**

(71) Applicant: Biomet Deutschland GmbH, 14167 Berlin (DE)
(72) Inventor: Richter, Anja, 14641 Wernitz (DE); Mannel, Henrich, 14532 Kleinmachnow (DE)
(74) Representative: Manitz, Finsterwald & Partner GbR

(57) **Abstract**

The present invention relates to a container for an implant (5), wherein the inner shape and/or dimensions of the container are adapted to mirror the outer shape and/or dimensions of the implant. The container can be used in methods for infection control and in methods for the diagnosis of infections of implants. These methods comprise an elution step of the microorganisms from an explanted implant, which is performed by ultrasonic methods using the entire container of the invention with the implant inside. Advantageously, the necessary volume of the solution for eluting the microorganisms form the surface of the implants can be reduced by up to 90 % due to the construction of the inside of the container. It will additionally enable a transport between the operating room (OR) and the lab without contamination risk.

## Description

### Field of the invention

The present invention relates to a container for an implant, characterized in that the inner shape and/or dimensions of the container are adapted to mirror the outer shape and/or dimensions of the implant. The invention further provides the use of the container in methods for infection control and in methods for the diagnosis of infections of implants. These methods comprise an elution step of the microorganisms from an explanted implant, which is performed by ultrasonic methods using the entire container of the invention with the implant inside. Advantageously, the necessary volume of the solution for eluting the microorganisms form the surface of the implants can be reduced by up to 90 % due to the construction of the inside of the container. It will additionally enable a transport between the operating room (OR) and the lab without contamination risk.

### Background of the invention

Infections of implants may occur during the implantation of a prosthesis, such as artificial joints and the like, into a subject, accompanied by long-lasting discomfort and chronic ailment after implantation. State of the art methods for the detection of pathogenic microorganisms directly from the joint include the aspiration of synovial fluid, a smear-test of the joint and of the surface of the prosthesis as well as the collection of periprosthetic tissue. According to currently valid standard methods for the diagnosis of prosthesis infections, only the detection of at least two identical pathogens from a joint was proving an infection. Therefore, in order to prove an infection always 3 to 6 tissue samples should be collected and analysed (see Berbari EF et al.: Risk factors for prosthetic joint infection: case-control study. Clin Infect Dis 1998; 27: 1247-1254). One goal of this procedure is to ensure that cross-contaminations, e.g. during explantation and handling the explanted prosthesis by the operating room (OR) staff, can be excluded. However, the aforementioned methods have originally not been developed for the diagnosis of prosthesis infections. Moreover, the sensitivity and specificity of these methods is unsatisfactory, because the associated microorganisms correspond to the normal flora of the skin, and thus a contamination of the sample cannot be excluded entirely. Another disadvantage of the aforementioned prior art methods is that only the tissue and the fluid surrounding an implant is analysed, but not the implant itself. However, in the adjacent tissue there can often not be found a substantial number of pathogens. Thus, the main goal of the present invention is to improve the sensitivity of the methods for diagnosing prosthetic infections, in particular to improve the identifiability of pathogenic microorganisms.

A prosthesis infection is usually caused by microorganisms that are attached to the surface of the implant itself, where the microorganisms form an extracellular matrix, the so-called biofilm. A biofilm facilitates the survival of the microorganisms, because the activity of the individual pathogens is decreased significantly. The presence of the bacteria within biofilm may make them reluctant to grow and inaccessible to antibiotic treatment. Microbial biofilms growing adherently on prosthetic surfaces may prevent the detection of the pathogens causing prosthetic joint infections. Bacteria contained in biofilms show an up to 1000-fold higher resistance to growth-dependent antimicrobial substances. This is confirmed by the fact that the minimum inhibitory concentration (MIC) and the minimal bactericidal concentration (MBC) in the biofilm is several times higher than for the free-living form of these microorganisms. There are estimates that biofilm-forming bacteria are responsible for about 60 % of all nosocomial infections and false-negative smear-tests obtained from wounds.

Thus, disrupting the biofilm from the prosthetic surface to release the pathogens into a test solution may improve the results of the diagnostic methods and thereby contribute to the treatment of the infection. To overcome the disadvantages of the conventional culture methods of periprosthetic tissue and/or fluid for the diagnosis of periprosthetic-joint infections, Trampuz et al. (Trampuz et al. 2007. Sonication of removed hip and knee prosthesis for diagnosis of infection. N Engl J Med 357:654-663) applied long-wave ultrasound to explanted prostheses in order to disrupt the biofilms and to enhance bacterial growth in subsequent microbiological analysis methods. Trampuz et al. reported that ultra-sound sonication before cultivation of explanted hip and knee prosthesis to dislodge adherent bacteria yielded a significant better recovery of bacterial growth and culture than the conventional methods using periprosthetic tissue and/or fluid.

Typically, when using an ultra sound method, the explanted prosthesis or components thereof are placed in a sterile container and covered with 400 ml or more of sterile Ringer's solution. This method has the disadvantage, that a high volume of the solution for the eluation of the microorganisms from the surface of the implant must be used. This is particularly disadvantageous for low grade infections which cannot be detected within these high volumes of the solution.

In another attempt, the explanted prostheses where placed into liquid-filled sterile plastic bags. However, through ultra-sound sonication, small holes occurred in the walls of the bags resulting an increased cross-contaminations accompanied by a false-high sensitivity and decreased specificity of the method.

### Description of the invention

It was the purpose of the invention to overcome the disadvantages of the prior art methods. In particular, it was the purpose of the invention to provide a device for diagnosing microbial infections of implants with high sensitivity and specificity, but a highly reduced risk of cross-contaminations.

This problem is solved by the provision of a container for an implant according to claim 1, characterised in that the inner shape and/or dimensions of the container are adapted to the outer shape and/or dimensions of the implant.

The container of the invention has the advantage, that the volume of the solution necessary for the destruction of the biofilm on the surface of the explanted implant and the elution of the microorganisms from the implant using ultra-sound sonication methods can be reduced drastically, e. g. from about 100 - 400 ml to 10 - 50 ml. In a preferred embodiment, the volume of said elution solution can be reduced by 50 %, 60 %, more preferably by 70 % or 80 %, most preferably by 90 % or more.

In general, the inner volume of the container of the invention is slightly larger than the volume of the explanted implant. Preferably, the inner volume of the container is 50 % or 40 % larger than the volume of the implant, more preferably 30 % or 20 % larger than the volume of the implant. Most preferably, the inner volume of the container is larger only minimally, e. g. 10 %, than the volume of the implant, and furthermore, the shape and/or dimensions of the inside of the container correspond to the outer shape and/or dimensions of the explanted implant, which is to be analysed for infections.

This embodiment has the advantage, that the container of the invention, when containing an explanted implant, must be filled with a minimum volume of an elution solution only. Moreover, it is possible to concentrate eluted microorganisms, including pathogenic bacteria, in a minimum volume of the elution solution. Thereby, an increase of the number of colony forming units (CFU) per ml elution solution can be reached, which in turn leads to a lower detection limit for the identification of specific microorganisms. In particular, local infections can now be detected with a much higher probability, in contrast to the conventional methods of the prior art, especially those, which are presently based on the use of ultra-sound.

Suitably, the container of the invention comprises a lid at the proximal end. In a preferred embodiment, such a lid is directly connected to the container via a connection means, and the lid and the container are made from one piece. The lid has the function that the container can be closed tightly and that the inner space of the container can be maintained sterile before an explanted implant is inserted into the container. After insertion of the explanted implant, the lid is especially suitable to close the container tightly in order to prevent any cross-contaminations entering from outside into the container.

In general, any types of lids are possible for use to seal the container. In a preferred embodiment, the lid of the container contains a means, e. g. a clip or else, to fix the implant inside the container after the lid is closed. This embodiment has the advantage that the implant inside the container is tightly fixed during performing the sonication method in the ultra-sound bath. Thereby, damages of the wall of the container due to a possibly moving implant inside the container during sonication in the ultra-sound bath can be prevented. This in turn reduces the risk of the occurrence of leakages in the container wall. In contrast to devices used in prior art methods, such as plastic bags, it is possible with the container of the invention to minimise, more preferably to exclude, the penetration of cross-contaminations through damages of the container body during sonication in the ultra-sound bath.

Moreover, due to the prevention of possible damages of the container during sonication in the ultra-sound bath, the safety of the OR staff and/or laboratory staff, who are handling the container of the invention, can be clearly improved compared to the means used in the prior art so far.

In a further embodiment, the container of the invention comprises an injection hole for the injection of the elution solution into the container, after the explanted implant has been inserted into the container and after the lid of the container has been sealed. Such an injection hole can be comprised in the container body itself or, more preferably, can form a part of the lid.

Any injection holes known from the prior art, which are suitable for methods performed under sterile conditions, are suitable for use with the container of the invention. Preferred are injection holes, which enable the administration of the elution solution to the inside of the container under sterile conditions. Suitably, the injection hole is sealed with a membrane, such as a rubber membrane or silicone membrane, which allows the application of the elution solution to the inside of the container with a syringe under sterile conditions.

In a preferred embodiment, the space between the explanted implant inserted into the container of the invention may be completely filled with an elution solution under sterile conditions. If the lid is sealed to the container and a syringe is used to administer the elution solution, the air contained in the sealed container will be replaced by the elution solution, i.e. the air contained in the sealed container must have the possibility to escape. This problem can be easily solved by placing the needle of a syringe in the membrane of the injections hole. Suitably, such a needle may be connected to a sterile filter or bacterial filter in order to allow the replaced air to escape from the container during administration of the elution solution without contaminating the lab environment with bacteria from the implant. Such bacterial filters are conventional and available in the art.

The container of the invention including the lid can be made of any suitable construction material, which allows either a sterile production of the container or the sterilisation of the container after the production. Accordingly, the container of the invention can be made of metal, glass or plastics or combinations thereof.

More preferably, the container of the invention is made of a plastics. Suitable plastics for producing the container of the invention are selected from those, which allow an easy and cheap manufacturing of the container on the one hand, but on the other hand are approved for the use in medicine or food industry and sustain ultrasonic treatment w/o weakening, i.e. w/o loss of stability.

Such plastics are suitably selected from thermo-formable plastics or plastics capable of injection moulding. Furthermore, the plastics used must not release substances, which are toxic for bacteria in order to prevent false-negative analysis results in the diagnostic methods of the invention.

Suitable plastics for use in the manufacturing of the container of the invention by injection moulding are for example selected from polyolefins such as polypropylene, acrylic glass, also known as plexiglass ((poly(methyl methacrylate), PMMA) for a transparent appearance, polystyrene (PS) and its copolymers (ABS = acrylonitrile-butadiene-styrene), polyamide (PA) and polyoxymethylene (POM).

In a preferred embodiment, the container of the invention is made of polypropylene.

Suitable thermo-formable plastics are any thermoplastics, e. g. those selected from polyvinylchloride (PVC), polyehtyleneterephthalate (PET), polystyrole (PS) and polyolefins, such as polypropylene, but also acrylnitrile-butadiene-styrole (ABS) or polyaryletherketone (PAEK) such as polyether ether ketone (PEEK).

In a further preferred embodiment, the container of the invention is made of polypropylene without addition of deforming supporting agents, plasticizers and biocides.

Generally, conventional methods known to the person skilled in the art, such as injection moulding or thermo-forming, are used to manufacture the container of the invention. Accordingly, the container of the invention is easily and cheap to produce.

In yet a further embodiment, the container of the invention may be re-used several times. In such cases, the container must be sterilized after each use. However, in order to increase the safety of the OR and lab staff when handling the container of the invention, a one-time-use of the container is intended. Most preferably, the container of the invention is used as a disposable.

It is possible, to produce a container of the invention, which is specifically adapted to any kind of implant. In other words, it is possible with the conventional production methods known in the art, to produce a container of the invention wherein at least the inner shape and/or dimensions of the container are adapted to the outer shape and/or dimensions of an implant selected from the group consisting of knee prostheses, hip prostheses, shoulder prostheses and the like.

In a further embodiment, the invention provides the use of the container of the invention in methods for infection control and/or diagnosis of infections of implants. These methods allow the handling of an explanted implant under sterile conditions. Cross-contaminations of the explanted implants can be minimized. There is a safe transport of the implant from the operating room (OR) to the laboratory, where the microbiological investigations are performed, possible. As described above, the use of the container of the invention in particular gains more precise results in the concentration-dependent analysis of infections of implants by limiting the volume of the elution solution.

The container of the invention can be used for quality control (QC) purposes and is also suitable to provide an easy way to dissolve bacteria adhered to an implant surface. The latter is of interest for septic revision surgery because the treatment regime depends on the specific strain identified. Therefore, the bacteria have to be detached from the implant and to be cultured using microbiological standard tests. The use of an ultrasonic bath can even improve the results. Nevertheless, accuracy is limited to the concentration as mentioned above.

Thus, in a further embodiment, the invention provides a method for controlling the infection of implants, such as prostheses or prosthetic components. Such method usually comprises in a first step the explanting of the prosthesis or prosthetic component. Thereafter, said prosthesis or prosthetic component is inserted into the container of the invention and the lid of the container is sealed tightly in order to prevent cross-contaminations of the explanted implant in the container, but also in order to prevent contaminations of the OR staff and the lab staff. The method further comprises the step of adding a sterile elution solution to the remaining space or volume between the inside of the container and the prosthesis or prosthetic component comprised in the container. Suitably, the sterile elution solution is inserted into the container under sterile conditions, for example using a syringe. This step can also be performed directly in the OR. Alternatively, the step of addition of the elution solution can also be performed later in the microbiology lab.

Preferably, the elution solution used in this method is a solution, which is not harmful for the microorganisms detached from the surface of the implant. A suitable elution solution may be selected from a buffer solution, an isotonic solution, culturing broth or else. More preferably, Ringer's solution, which is isotonic with blood, is used as elution solution in the container of the invention.

Thereafter, the container of the invention containing the prosthesis or prosthetic component and the elution solution inside, is placed into an ultrasonic bath and sonicated for a desired time. After sonication, aliquot samples can be taken from the container under sterile conditions. Thereby, it is not necessary to open the container. The samples can be taken from the container via the same way, e. g. by using a sterile syringe, as was used to add the elution solution. Again, this possibility to obtain samples from the elution solution without opening the container minimizes the risk for cross-contaminations of the samples on the one hand, and of the laboratory personal on the other hand.

The aliquot samples taken from the elution solution in the container of the invention may then be analysed for microbial infections, using any available conventional methods, such as plating the samples on different agar plates, e.g. aerobic and anaerobic sheep-blood agar plates, and calculating the number of colony forming units (CFU) after cultivation.

By using the container of the invention in the methods for controlling an infection of an implant and/or for the diagnosing of an infection of an implant, it is possible to reduce the necessary volume of the elution solution to a minimum volume. The sonication step leads to the decomposition of the biofilms that are established on the surface of said prostheses or prosthetic components, and further to the elution of the microorganisms from the surface of the prostheses or prosthetic components. Due to the minimum volume of the elution solution used, the eluted microorganisms are concentrated in the elution solution of the container of the invention rather than they are diluted in this solution as it is known from the devices presently used in the prior art.

In a preferred embodiment, the invention provides a method for controlling the infection of prostheses or prosthetic components comprising the steps of
a) explanting a prosthesis or prosthetic component,
b) inserting said a prosthesis or prosthetic component into the container of the present invention;
c) sealing the lid of the container,
d) adding a sterile elution solution to the space or volume between the inside of the container and said prosthesis or prosthetic component,
e) sonicating the container together with said prosthesis or prosthetic component and the elution solution inside in an ultrasonic bath,
f) taking aliquot samples from the container under sterile conditions, and
g) analysing the aliquot samples for microbial infections.

The volume of the aliquot samples taken from the container after sonication can vary, dependent on the type of microbiological analysis or test, which is performed subsequently. Usually, the volume of the aliquot samples is between 0.05 and 0.5 ml. Typically, the volume of the aliquot samples is 0.1 ml or 0.5 ml.

In jet a further embodiment, the method for controlling the infection of an implant, such as prostheses or prosthetic components, can also be performed with implants that are already removed from the body of the subject, i. e. which are already explanted.

Accordingly, the present invention further provides a method for controlling the infection of an already explanted prosthesis or prosthetic component, comprising the steps of
a) inserting said a prosthesis or prosthetic component into the container of the invention;
b) sealing the lid of the container,
c) adding a sterile elution solution to the remaining space or volume between the inside of the container and said prosthesis or prosthetic component,
d) sonicating the container together with said prosthesis or prosthetic component and the elution solution inside in an ultrasonic bath,
e) taking aliquot samples volume from the container under sterile conditions,
   and
f) analysing the aliquot samples for microbial infections.

The method of the diagnosis of microbiological infections of an implant in a subject according to the invention is further suitable for the identification of specific bacteria.

Besides the aforementioned conventional methods for the identification of microorganisms, in particular bacteria, more particular pathogenic bacteria, it is also possible to use other methods for the identification of bacteria. Such methods may be selected from polymerase chain reaction (PCR), IR spectroscopy, microarray based bacteria identification and the like. The aforementioned methods for controlling the infection of implants, such as prostheses or prosthetic components, may thus further comprise a step of identifying and distinguishing bacterial strains using PCR, IR spectroscopy or microarrays.

The most common pathogens in prosthesis and implant infections are *Staphylococcus aureus, Staphylococcus epidermidis* and other *Staphylococcus* spp., *Corynebacterium* spp., *Streptococcus* spp., *Enterococcus* spp., *Propionibacterium* spp., etc.

The colony count of these pathogens in samples, when taken from periprosthetic tissue, is often so low that these bacteria cannot be detected within these samples. The same holds true for the ultrasound methods presently used in the prior art, where a very high volume of elution solution is used. Only with the container and methods of the present invention, wherein a minimum volume of the elution solution is used during ultra-sound sonication, it is possible to detect and diagnose low-grade infections of said pathogens with high accuracy and reliability. The container of the invention and the methods of the invention using said container are thus suitable for the detection and diagnosis of low-grade infections with highly problematic pathogens, e.g. those selected from the group consisting of *Staphylococcus aureus, Staphylococcus epidermidis* and other *Staphylococcus* spp., *Corynebacterium* spp., *Streptococcus* spp., *Enterococcus* spp., *Propionibacterium* spp., etc.

The invention is further illustrated and specified by three figures and one example.

### Brief description of the figures

Figure 1 shows a container of the invention capable of harbouring an explanted hip prostheses.
Figure 2 shows a cross-section through a container of the invention, containing an explanted hip prostheses.
Figure 3 shows a container of the invention capable of harbouring an explanted plate prosthesis used in fracture treatment.
Figure 4 shows a cross-section through a container of the invention, containing an explanted plate prosthesis used in fracture treatment.
Figure 5 shows a top view on the lid of the container of the invention.

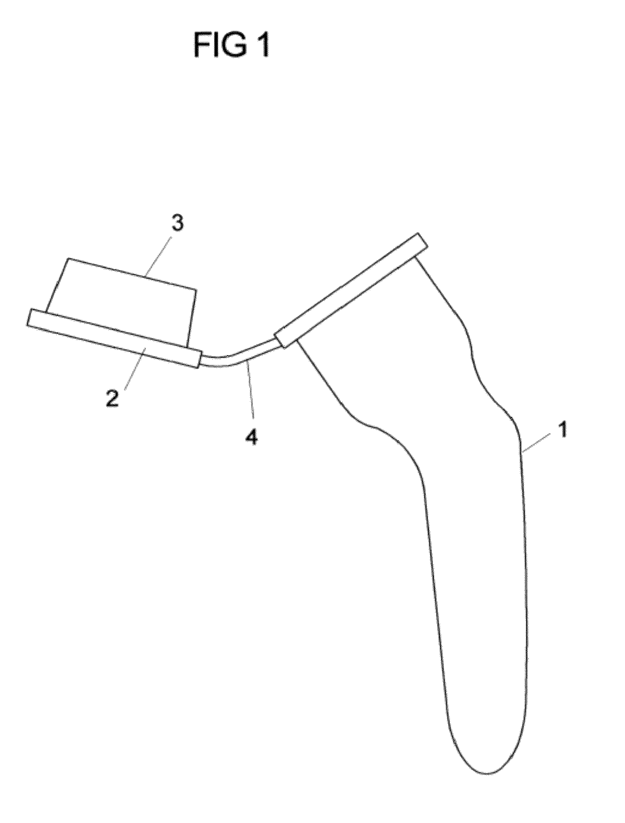

The shape and dimensions of the container shown in figure 1 are adapted to the shape and dimensions of a hip prostheses. The container is made of polypropylene. The container comprises a container body 1, in which the explanted hip prostheses can be inserted, a lid 2 comprising a clip 3 for sealing the container body 1 with the lid 2. The lid 2 and the container body 1 are connected via a plastic strip 4 as the connection means, which is also made of polypropylene. The container body 1, the lid 2 comprising a clip 3 and the plastic strip 4 are formed as one piece.

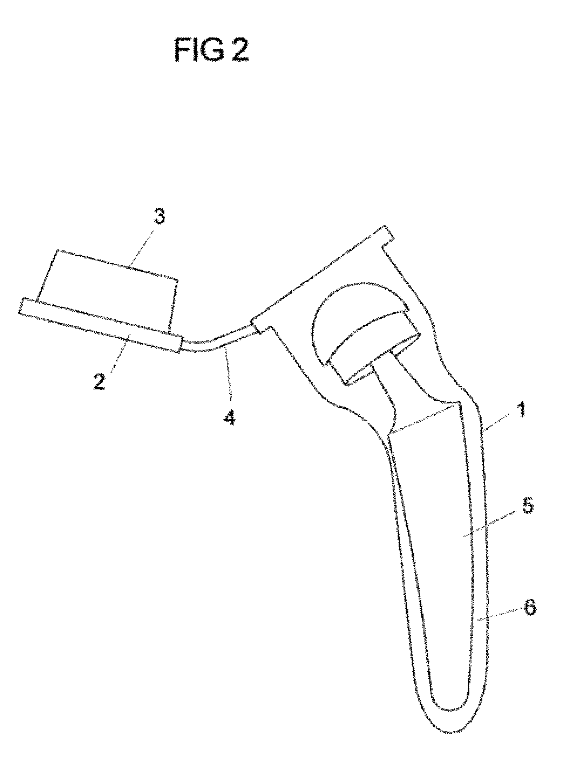

Figure 2 shows the container of the invention, when it is in use. An explanted hip prostheses 5 was inserted into the container body 1. It can be seen that the free volume 6 between the explanted hip prostheses 5 and the inside wall of the container body 1 is very small. When the lid 2 is sealed to the container body 1, the clip 3 will fix the explanted hip prostheses 5, especially the proximal end of the hip prostheses 5, inside the container.

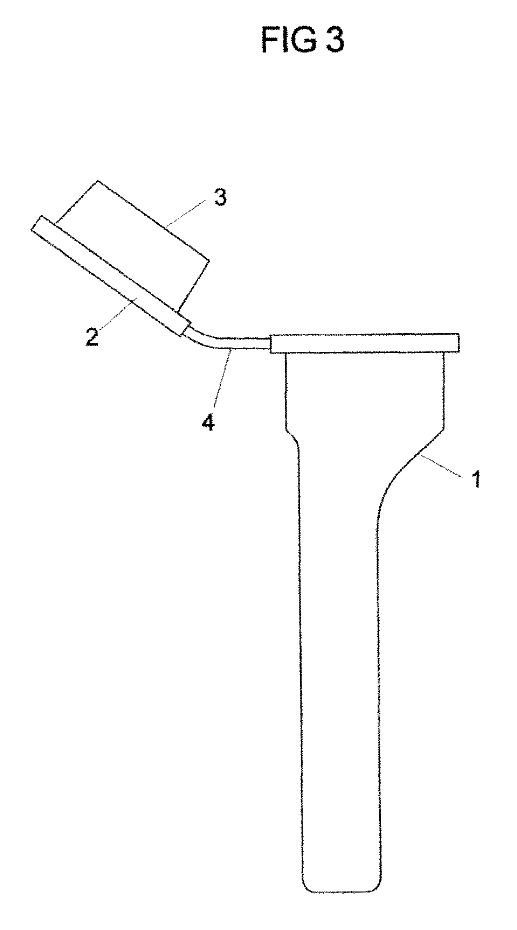

The shape and dimensions of the container shown in figure 3 are adapted to the shape and dimensions of a plate prostheses as used in the treatment of trauma, in particular the treatment of fractures. The container is made of polypropylene. The container comprises a container body 1, in which the explanted plate prostheses can be inserted, a lid 2 comprising a clip 3 for sealing the lid 2 to the container body 1. The lid 2 and the container body 1 are connected via a plastic strip 4 as the connection means, which is also made of polypropylene. The container body 1, the lid 2 comprising a clip 3 and the plastic strip 4 are formed as one piece.

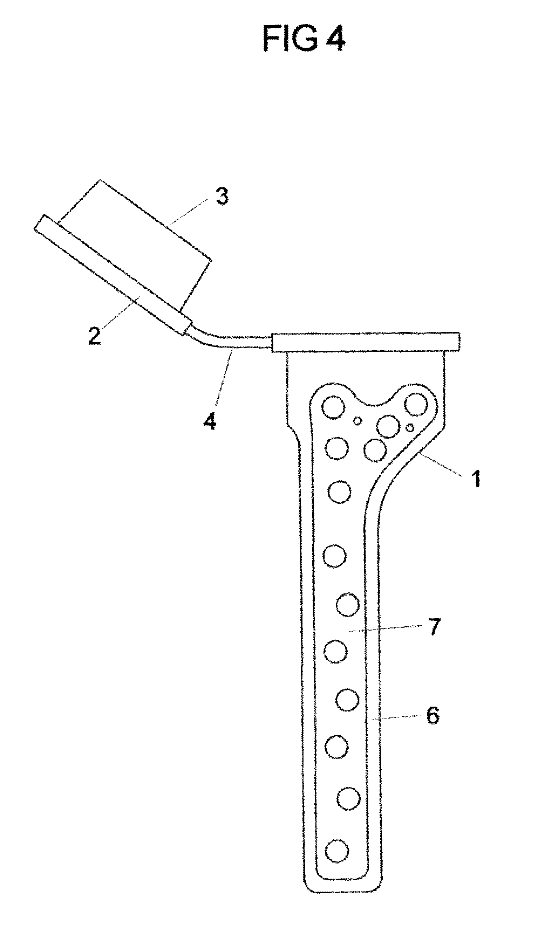

Figure 4 shows the container of the invention, when it is in use. An explanted plate prostheses 7 was inserted into the container body 1. It can be seen that the free volume 6 between the explanted plate prostheses 7 and the inside wall of the container body 1 is very small. When the lid 2 is sealed to the container body 1, the clip 3 will fix the explanted plate prostheses 7, especially the proximal end of the plate prostheses 7, inside the container.

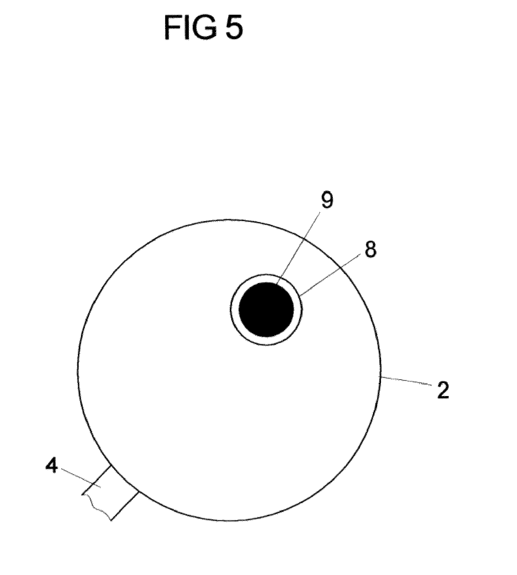

Figure 5 shows a top view on the lid 2 of the container of the invention from outside. The lid 2 contains an injection hole 8, which comprises a rubber membrane 9 for injection of the elution solution into the container under sterile conditions, e.g. by using a syringe.

### Examples of the invention

### Example 1: Microbiological diagnosis of an explanted hip prosthesis

An explanted hip prostheses was placed in a container comprising an inner shape and inner dimensions, which were adapted to the outside shape and dimensions of the hip prostheses during manufacturing by injection moulding. After insertion of the hip prostheses, the lid 2 was sealed to the container body 1. Thereafter, the container was completely filled with Ringer's solution using a sterile syringe. 10 ml Ringer's solution were necessary in order to fill the free space between the explanted hip prostheses 5 and the inside wall of the container body 1.

The container was vortexed for 30 seconds using a vortex-mixer, and then subjected to sonication (frequency, 40 ± 2 kHz; and power density 0.22 ± 0.04 W/cm², in an aqua sonic model 750 T ultrasound bath (VWR scientific products)) for 5 min, followed by additional vortexing for 30 seconds. The resulting sonicated fluid was removed under sterile conditions with a sterile syringe from the container without opening the container. The sonicated fluid was plated in 0.5 ml aliquots onto aerobic and anaerobic sheep-blood agar plates

For comparison, synovial fluid was obtained from a subject and inoculated in 0.1 ml aliquots onto aerobic and anaerobic shape-blood agar plates (BD diagnostic systems).

The aerobic and anaerobic sheep-blood agar plates from both sample types were incubated at 35 to 37° Celsius in 5 to 7 % carbon dioxide aerobically and anaerobically for 5 days and 7 days, respectively.

Always 6 parallel samples were analysed from both sample groups, synovial fluid samples and sonication samples, respectively.

The results are shown in table 1:
Table 1: Distribution of pathogens in sonication culture (SC) and synovial fluid (SF) Sample # / SC SC SC SC SC SC SF SF SF SF SF SF Pathogens 1 2 3 4 5 6 1 2 3 4 5 6 (CFU/ml) *S. aureus* 3 2 4 2 3 3 0 1 0 0 1 1 *S. epidermidis* 4 6 6 5 3 4 1 0 0 2 1 2 *Streptococcus* 8 7 4 7 8 6 2 1 0 2 0 0 spp. *Enterococcus* 6 6 5 6 5 5 0 0 0 0 0 1 spp. *Corynebacteri* 5 4 4 3 4 4 1 1 1 0 1 0 um spp. *Propioni-* 8 10 9 7 10 8 2 2 3 1 0 2 *bacterium* acnes *Fusobacterium* 3 5 3 4 4 5 0 1 2 1 1 0 spp.

The above results show that with the container of the present invention, a concentration of pathogens eluted from an explanted hip prostheses occurs. Some of the pathogens are either not detectable in synovial fluid or are detectable but without reliability.

In contrast, due to the concentration effect achieved by using the container of the present invention in combination with ultrasound techniques, it is possible to detect and diagnose pathogenic infections in almost each sample with high reliability.

### List of reference numerals

- 1: container body
- 2: lid
- 3: means for fixing the implant inside the container
- 4: means for connecting lid and container body
- 5: hip prostheses
- 6: space between hip prostheses and inner wall of container body
- 7: plate prosthesis
- 8: injection hole
- 9: membrane

## Claims

1. A container for an implant, **characterized in that** the inner shape and/or dimensions and/or volume of the container are adapted to the outer shape and/or dimensions and/or volume of the implant.

2. The container according to claim 1, **characterized in that** the inner volume of the container is slightly larger, e.g. 30 % to 10 % larger than the outer volume of the implant.

3. The container according to claim 1 or 2, wherein the container comprises a lid at the proximal end.

4. The container according to claim 3, wherein the lid comprises a means to fix the implant inside the container.

5. The container according to any one of claims 1 to 4, further comprising an injection hole and/or a membrane.

6. The container according to any one of claims 1 to 5, wherein the container consists of a thermo-formable plastics or a plastics capable of injection moulding.

7. The container according to any one of claims 1 to 6, wherein the container consists of:
a plastics capable of injection moulding selected from the group consisting of polyolefins such as polypropylene, acrylic glass, polystyrene (PS) and/or its copolymers (e.g. ABS = acrylonitrile-butadiene-styrene), polyamide (PA) and polyoxymethylene (POM)
or
a thermo-formable plastics selected from the group consisting of polyvinylchloride (PVC), polyehtyleneterephthalate (PET), polystyrole (PS), polyolefins, such as polypropylene, acrylnitrile-butadiene-styrole (ABS) or polyaryletherketone (PAEK) such as polyether ether ketone (PEEK).

8. The container according to any one of claims 1 to 7, wherein the container consist of a plastics, preferably polypropylene, without addition of deforming supporting agents, plasticizers and biocides.

9. A method of manufacturing the container according to any one of claims 1 to 10, comprising thermo-forming or injection moulding.

10. A method for controlling the infection of prostheses or prosthetic components comprising the steps of:
a) explanting a prosthesis or prosthetic component,
b) inserting said prosthesis or prosthetic component into the container according to any of claims 1 to 10;
c) sealing the lid of the container according to any one of claims 1 to 10,
d) adding a sterile elution solution to the space or volume between the inside of the container and said prosthesis or prosthetic component,
e) sonicating the container together with said prosthesis or prosthetic component and the elution solution inside in an ultrasonic bath,
f) taking aliquot samples volume from the container under sterile conditions,
g) analyzing the aliquot samples for microbial infections.

11. The method of claim 10, wherein an already explanted prosthesis or prosthetic component is used.

12. A method for the diagnosis of microbial infections in a subject, comprising the steps of the method of claim 10 or 11 and further comprising the step of identifying specific bacterial strains.

13. The method of claim 12, wherein said specific bacterial strains are selected from *Staphylococcus aureus, Staphylococcus epidermidis* and other *Staphylococcus spp., Corynebacterium* spp., *Streptococcus spp., Enterococcus* spp. and *Propionibacterium* spp..

14. Use of the container according to any one of claims 1 to 8 in a method for infection control of implants or a method for the diagnosis of microbial infections according to any one of claims 10 to 13 or for septic revision surgery.

15. The container, use or method according to any one of the preceding claims, wherein the prosthesis or prosthetic component is selected from the group consisting of knee prostheses, hip prostheses, shoulder prostheses, artificial joints, limb salvage, trauma nails, screws, plates etc.
